# EUROPEAN PATENT SPECIFICATION

(11) **EP 2 679 671 B1**
(45) Date of publication and mention of the grant of the patent: **11.12.2019**
(21) Application number: 13186524.8
(22) Date of filing: 27.05.2003
(51) Int. Cl.: C12N 5/0735, C12N 15/873, A61K 35/12

(54) **A bank of stem cells for producing cells for transplantation having HLA antigens matching those of transplant recipients, and methods for making and using such a stem cell bank**
Eine Bank von Stammzellen zur Herstellung von Zellen für die Transplantation, die HLA-Antigene hat, die mit denen von Transplantationspatienten passen, und Verfahren zur Herstellung und Verwendung einer solchen Stammzell-Bank
Une banque de cellules souches pour produire des cellules pour la transplantation ayant des antigènes HLA correspondant à ceux des greffés et des méthodes de fabrication et d'utilisation d'une telle banque de cellules souches

(30) Priority: 24.05.2002 US 382616 P; 21.02.2003 US 448585 P
(43) Date of publication of application: 01.01.2014
(62) Divisional of application: 03736723.2
(73) Proprietor: Advanced Cell Technology, Inc., Worcester, MA 01605 (US)
(72) Inventor: West, Michael D., Worchester, MA 01605 (US)
(74) Representative: Daniels, Jeffrey Nicholas

(56) References cited:
- WO-A1-01/30978
- WO-A1-01/32015
- MITALIPOV S M ET AL: "PARTHENOGENETIC ACTIVATION OF RHESUS MONKEY OOCYTES AND RECONSTRUCTED EMBRYOS", BIOLOGY OF REPRODUCTION, NEW YORK, NY [U.A.] : ACADEM. PRESS, US, vol. 65, no. 1, 1 July 2001 (2001-07-01), pages 253-259, XP001117868, ISSN: 0006-3363, DOI: 10.1095/BIOLREPROD65.1.253
- CIBELLI JOSE B ET AL: "Parthenogenetic Stem Cells in Nonhuman Primates", SCIENCE, AMERICAN ASSOCIATION FOR THE ADVANCEMENT OF SCIENCE, WASHINGTON, DC; US, vol. 295, no. 5556, 1 February 2002 (2002-02-01), page 819, XP008091029, ISSN: 0036-8075, DOI: 10.1126/SCIENCE.1065637
- VAN DOORNINCK J H ET AL: "A MOUSE MODEL FOR THE CYSTIC FIBROSIS DELTA-F508", EMBO JOURNAL, OXFORD UNIVERSITY PRESS, SURREY, GB, vol. 14, no. 18, 1 January 1995 (1995-01-01), pages 4403-4411, XP008069832, ISSN: 0261-4189
- KIM KITAI ET AL: "Histocompatible embryonic stem cells by parthenogenesis", SCIENCE, AMERICAN ASSOCIATION FOR THE ADVANCEMENT OF SCIENCE, WASHINGTON, DC; US, vol. 315, no. 5811, 26 January 2007 (2007-01-26), pages 482-486, XP002467552, ISSN: 0036-8075, DOI: 10.1126/SCIENCE.1133542

## Description

The invention described herein relates to stem cell lines that are parthenogenetically-derived and which are homozygous for at least one disease-causing allele.

Also described herein are methods for producing a collection of human and non-human stem cell cultures, preferably human stem cell cultures, each of which contains pluripotent stem cells that have genes encoding the same set of critical cell surface antigenic proteins, e.g., histocompatibility antigens(e.g., HLA antigens in the case of human) as are present on the cells of members of a human population. (By critical antigens is meant the set of antigens that form the major histocompatibility complex and other antigens such as blood group antigens that are involved in immuno-mediated rejection when collogenic cells and tissues are transplanted into donors that express a different set of histocompatibility and other critical antigens). The methods disclosed herein include deriving such human stem cell cultures from cells of early embryos produced by parthenogenesis.

The description also relates to methods wherein such human and non-human stem cell cultures are induced to differentiate ex or in vivo into cell types that are useful for therapeutic cell transplantation; and to methods by which the differentiated cells are isolated from other cell types. The description also relates to methods in which stem cell-derived differentiated cells having a selected set of critical cell surface antigens are therapeutically transplanted or engrafted to a recipient, e.g., a human patient in need of a cell transplant having cells that express the same critical cell surface antigens. The description further relates to a collection or "bank" of cultures of different types of stem cells, each culture having a different set of genes encoding cell surface antigenic proteins present in a human population; to compositions comprising the individual stem cell cultures that make up such a stem cell bank; and to compositions comprising differentiated cells derived from such stem cells.

Preferably, stem cell banks produced according to the description will comprise stem cell lines which are homozygous for MHC alleles which occur very frequently in the human population. Typically, a stem cell bank according to the description will comprise at least 15 stem cell lines and more preferably at least 100 to 1000 stem cell lines. Thereby, the stem cell bank will provide maximal therapeutic and diagnostic efficacy as it will contain cells that are histocompatible for a wide range of potential transplant recipients.

### BACKGROUND OF THE INVENTION

### A. Histocompatibility and Transplant Rejection:

Histocompatibility is a largely unsolved problem in transplant medicine. Rejection of transplanted tissue is the result of an adaptive immune response to alloantigens on the grafted tissue by the transplant recipient. The alloantigens are "non-self" proteins, i.e., antigenic proteins that vary among individuals in the population and are identified as foreign by the immune system of a transplant recipient. The antigens on the surfaces of transplanted tissue that most strongly evoke rejection are the blood group (ABO) antigens and the major histocompatibity complex (MHC) proteins and in the case of humans, the human leukocyte antigen (HLA) proteins.

The blood group antigens were first described by Landsteiner in 1900; they are branched oligosaccharides that are attached to proteins and lipids on the surfaces of red blood cells, endothelial cells, and other cells, and are also present in secretions such as saliva. Compatibility of the blood group antigens of the ABO system of a vascularized organ or tissue transplant with those of the transplant recipient is generally required; but blood group compatibility may be unnecessary for many types of cell transplants.

The HLA proteins are encoded by clusters of genes that form a region located on chromosome 6 known as the Major Histocompatibility Complex, or MHC, in recognition of the important role of the proteins encoded by the MHC loci in graft rejection. Accordingly, the HLA proteins are also referred to as MHC proteins. The MHC genes and proteins will be used interchangeably in this application as the application encompasses human and non-human animal applications. The HLA or MHC proteins normally play a role in defending the body against foreign pathogens such as viruses, bacteria, and toxins. They are cell surface glycoproteins that bind peptides at intracellular locations and deliver them to the cell surface, where the combined ligand is recognized by a T cell. Class I MHC proteins are found on virtually all of the nucleated cells of the body. The class I MHC proteins bind peptides present in the cytosol and form peptide-MHC protein complexes that are presented at the cell surface, where they are recognized by cytotoxic CD8+ T cells. Class II MHC proteins are usually found only on antigen-presenting cells such as B lymphocytes, macrophages, and dendritic cells. The class II MHC proteins bind peptides present in a cell's vesicular system and form peptide-MHC protein complexes that are presented at the cell surface, where they are recognized by CD4+ T cells. CD4+ T cells activated by class II MHC proteins undergo clonal expansion with production of regulatory cytokines that signal helper and cytotoxic T cells. Unfortunately for those in need of transplants, the frequency of T cells in the body that are' specific for non-self MHC molecules is relatively high, with the result that differences at MHC loci are the most potent critical elicitors of rejection of initial grafts. Rejection of most transplanted tissues is triggered predominantly by the recognition of class I MHC proteins as non-self proteins. T cell recognition of foreign antigens on the transplanted tissue sets in motion a chain of signaling and regulatory events that causes the activation and recruitment of additional T cells and other cytotoxic cells, and culminates in the destruction of the transplanted tissue. (Charles A. Janeway et al., Immunobiology, Garland Publishing, New York, NY, 2001, p. 524).

### B. The Genes Encoding MHC Proteins:

The MHC genes are polygenic - each individual possesses multiple, different MHC class I and MHC class II genes. The MHC genes are also polymorphic - many variants of each gene are present in the human and non-human population. In fact, the MHC genes are the most polymorphic genes known. Each MHC Class I receptor consists of a variable *α* chain and a relatively conserved *β*2-microglobulin chain. Three different, highly polymorphic class I *α* chain genes have been identified. These are called HLA-A, HLA-B, and HLA-C. Variations in the *α* chain chains account for all of the different class I MHC genes in the population. MHC Class II receptors are also made up of two polypeptide chains, an *α* chain and a *β* chain, both of which are polymorphic. In humans, there are three pairs of MHC class II *α* and *β* chain genes, called HLA-DR, HLA-DP, and HLA-DQ. Frequently, the HLA-DR cluster contains an extra gene encoding a *β* chain that can combine with the DR *α* chain; thus, an individual's three MHC Class II genes can give rise to four different MHC Class II molecules.

In humans, the genes encoding the MHC class I *α* chains and the MHC class II *α* and *β* chain are clustered on the short arm of chromosome 6 in a region that extends over from 4 to 7 million base pairs that is called the major histocompatibility complex. Every person usually inherits a copy of each HLA gene from each parent. If an individual's two alleles for a particular MHC locus encode structurally different proteins, the individual is heterozygous for that MHC allele. If an individual has two MHC alleles that encode the same MHC molecule, the individual is homozygous for that MHC allele. Because there are so many different variants of the MHC alleles in the population, most people have heterozygous MHC alleles. The numbers of different alleles found for each type of MHC class I *α* chain and MHC class II *α* and *β* chains as of January 2003 are shown in Table 1.

**TABLE 1. The numbers of different alleles for the polymorphic MHC class I and class II chains identified as of January, 2003.**

| MHC chain | no. of alleles |
|---|---|
| HLA-A | 266 |
| HLA-B | 511 |
| HLA-C | 6 |
| HLA-DRA | 3 |
| HLA-DRB | 403 |
| HLA-DQA1 | 23 |
| HLA-DQB1 | 53 |
| HLA-DPA1 | 20 |
| HLA-DPB1 | 101 |

The data in Table 1 is from the Internet web site of the Informatics Group of the Anthony Nolan Trust, The Royal Free Hospital, Hampstead, London, England. Lists of identified HLA Class I and Class II alleles are also available at the same web site.

### C. Matching MHC Types to Inhibit Rejection of Transplants:

Since the recognition that non-self MHC molecules are a major determinant of graft rejection, much effort has been put into developing assays to identify the MHC types present on the cells of tissue to be transplanted, and on the cells of transplant recipients, in order to match the types of MHC molecules present in the transplant tissue with those of the recipient. Tissue typing, the detection of MHC antigens, is performed by various means; for example, (i) by serology, using antibodies specific for particular MHC molecules to detect the presence of the targeted MHC molecules on donor or recipient cells, e.g., by the lymphocytotoxicity test; (ii) by detection of antibodies of a transplant recipient that bind specifically to a MHC protein of transplant tissue; and (iii) by direct analysis of the nucleotide sequence of the DNA of the MHC alleles. Most tissue typing for organ banking purposes is done by determining the blood type (ABO typing) and by typing the patient's and donor cells using serological methods; however, the use of rapid and reliable DNA-specific methods is increasing. Such methods can employ sequence-specific oligonucleotide primers and amplification by the polymerase chain reaction (PCR), and can be augmented by combining fluorescent detection methods with the use of a DNA chip to which are bound sequence specific oligonucleotides designed to detect unique sequences present in the different MHC alleles.

At present, tissue typing to match the HLA antigens of a transplant with those of a recipient is usually limited to the Class I HLA-A and -B antigens, and the Class II HLA-DR antigens. Most transplant donors are unrelated to the transplant recipient, and finding a tissue type to match that of the recipient usually involves matching the blood type and as many as possible of the 6 HLA alleles - two for each HLA-A, -B, and -DR locus. Transplant centers do not usually consider potential incompatibilites at other HLA loci, such as HLA-C and HLA-DPB1, although mismatches at these loci can also contribute to rejection. Considering only the combinations of maternal and paternal alleles of the HLA-A, HLA-B, and HLA-DR loci identified to date, there is a complexity of billions of possible tissue types. The task of matching HLA types of organs for transplant is simplified in that HLA-A and HLA-B are usually identified serologically. The number of HLA antigens identified serologically is considerably less than the number of different HLA antigens based on DNA sequencing. The World Health Organization (WHO) has recognized 28 distinct antigens in the HLA-A locus and 59 in the HLA-B locus, based on serological typing. Matching organs is also simplified to some extent by the fact that some alleles are much more common than others. Some of the more common HLA-A and HLA-B alleles are shown in Table 2:

**Table 2. Frequency of common HLA-A and HLA-B alleles in the population.**

| HLA-A | (Frequency (%)) | HLA-B | (Frequency (%)) |
|---|---|---|---|
| HLA-A1 | (25.1) | HLA-B5 | (15.2) |
| HLA-A2 | (44.8) | HLA-B7 | (18.2) |
| HLA-A3 | (22.6) | HLA-B8 | (16.7) |
| HLA-A24 | (18.2) | HLA-B12 | (32.5) |
| HLA-A11 | (11.8) | HLA-B14 | (8.8) |
| HLA-A28 | (9.8) | HLA-B 18 | (11.3) |
| HLA-A29 | (10.3) | HLA-B35 | (15.2) |
| HLA-A32 | (9.8) | HLA-B40 | (13.7) |
| HLA-B15 | (12.3) | | |

(from Snell GD et al, Histocompatibility, New York, Academic Press, 1976)

The frequencies with which the various alleles appear in a population is not random; it depends on the racial makeup of the population. Dr. Motomi Mori has determined the frequencies with which thousand of different haplotypes of HLA-A, -B, and -DR loci appear in Caucasian, African-American, Asian-American, and Native American populations. Each haplotype is a particular combination of HLA-A, HLA-B, and HLA-DR loci that is present on a single copy of chromosome no. 6. The frequencies of several relatively common HLA-A, -B, and -DR haplotypes are shown in Table 3 to illustrate the wide variation in HLA haplotype frequencies in some of the racial groups that make up the North American population. In interpreting haplotype frequency data such as that shown in Table 3, one must bear in mind that cells of patients and organs are diploid and have an HLA type that is the product of the HLA haplotypes of the chromosomes inherited from both parents.

**Table 3. Examples of HLA-A, -B, -DR haplotype frequencies HLA-A, -B, and -DR haplotype frequencies (expressed in percent) and their respective rankings within each racial group: Caucasian (CAU), African-American (AFR), Asian-American (ASI) and Native American (NAT).**

| Haplotype | | | | Frequency (%) | | | | Ranking | | | | |
|---|---|---|---|---|---|---|---|---|---|---|---|---|
| A | B | DR | CAU | AFR | ASI | LAT | NAT | CAU | AFR | ASI | LAT | NAT |
| 1 | 7 | 2 | 0.5349 | 0.2094 | 0.0798 | 0.1888 | 0.2812 | 21 | 58 | 262 | 91 | 62 |
| 1 | 8 | 3 | 5.1812 | 1.2491 | 0.3195 | 1.6733 | 4.7439 | 1 | 2 | 54 | 3 | 1 |
| 2 | 14 | 1 | 0.1563 | 0.0444 | 0.0076 | 0.3794 | 0.0624 | 107 | 539 | 1451 | 39 | 312 |
| 2 | 35 | 4 | 0.1457 | 0.0737 | 0.3293 | 1.2858 | 0.6342 | 115 | 302 | 49 | 4 | 12 |
| 2 | 35 | 8 | 0.0823 | 0.0931 | 0.1756 | 1.7641 | 0.3289 | 241 | 226 | 122 | 1 | 46 |
| 2 | 44 | 4 | 2.1507 | 0.6506 | 0.1276 | 0.6906 | 2.0004 | 3 | 4 | 170 | 12 | 3 |
| 3 | 7 | 2 | 2.6285 | 0.7596 | 0.1891 | 1.1986 | 2.7083 | 2 | 3 | 113 | 5 | 2 |
| 3 | 7 | 4 | 0.4411 | 0.1534 | 0.0498 | 0.1795 | 0.4448 | 30 | 104 | 408 | 98 | 29 |
| 3 | 7 | 8 | 0.0848 | 0.0367 | 0.0000 | 0.0622 | 0.0537 | 230 | 653 | 14053 | 310 | 366 |
| 3 | 35 | 1 | 1.0224 | 0.2741 | 0.1372 | 0.3552 | 0.8125 | 7 | 29 | 156 | 44 | 8 |
| 31 | 51 | 4 | 0.0915 | 0.0342 | 0.1646 | 0.2597 | 0.5691 | 209 | 699 | 135 | 64 | 16 |
| 32 | 14 | 7 | 0.2617 | 0.0513 | 0.0046 | 0.1324 | 0.1775 | 57 | 479 | 1858 | 140 | 104 |

The data in Table 3 was produced for The National Marrow Donor Program Donor Registry, and is available at the Internet web site of Motomi Mori, Ph.D., Huntsman Cancer Institute, Salt Lake City, Utah.

### D. Rejection Triggered by Minor Histocompatibility Antigens:

Matching the MHC molecules of a transplant to those of the recipient significantly improves the success rate of clinical transplantation; however, it does not prevent rejection, even when the transplant is between HLA-identical siblings. This is because rejection is also triggered by differences between the minor histocompatibility antigens. These polymorphic antigens are actually "non-self" peptides bound to MHC molecules on the cells of the transplant tissue. The rejection response evoked by a single minor histocompatibility antigen is much weaker than that evoked by differences in MHC antigens, because the frequency of the responding T cells is much lower (Janeway et al., supra, page 525). Nonetheless, differences between minor histocompatibility antigens will often cause the immune system of a transplant recipient to eventually reject a transplant, even where there is a match between the MHC antigens, unless immunosuppressive drugs are used.

### E. Inadequate Supply of Cells, Tissues, and Organs for Transplant.

The number of people in need of cell, tissue, and organ transplants is far greater than the available supply of cells, tissues, and organs suitable for transplantation. Under these circumstances, it is not surprising that obtaining a good match between the MHC proteins of a recipient and those of the transplant is frequently impossible, and many transplant recipients must wait for an MHC-matched transplant to become available, or accept a transplant that is not MHC-matched. If the latter is necessary, the transplant recipient must rely on heavier doses of immunosuppressive drugs and face a greater risk of rejection than would be the case if MHC matching had been possible. There is presently a great need for new sources of cells, tissues, and organs suitable for transplantation that are histocompatible with the patients in need of such transplants.

WO 01/30978 A1 describes gynogenetic or androgenetic production of pluripotent cells and cell lines, and use thereof to produce differentiated cells and tissues.

Mitalipov et al., describes parthenogenetic activation of rhesus monkey oocytes and reconstructed embryos (Biology of Reproduction (2001); 65(1): 253-259).

WO01/32015 A1 describes the use of haploid genomes for genetic diagnosis, modification and multiplication.

Cibelli et al., describes parthenogenetic stem cells in nonhuman primates (Science (2002); 295(5556): 819).

Van Doorninck et al., describes a mouse model for the cystic fibrosis ΔF508 mutation (EMBO Journal (1995); 14(18): 4403-4411).

The present invention provides a pluripotent stem cell line homozygous for at least one recessive disease-causing allele that is parthenogenetically-derived produced by the method comprising:
a) activating unfertilized oocytes whose DNA contains a recessive disease-causing allele;
b) exposing the activated oocytes to chemical treatment that inhibits extrusion of the second polar body;
c) culturing the exposed oocytes from step (b) in vitro under conditions resulting in formation of a blastocyst;
d) culturing inner cell mass cells of the blastocyst in vitro under conditions resulting in production of stem cells; and
e) screening the stem cells to identify stem cells that are homozygous for a recessive disease-causing allele, thereby obtaining a
   homozygous pluripotent stem cell for at least one recessive disease-causing allele.

The present invention also provides a method for producing pluripotent stem cells homozygous for at least one recessive disease-causing allele, comprising:
a) activating unfertilized oocytes whose DNA contains a recessive disease-causing allele;
b) exposing the activated oocytes to chemical treatment that inhibits extrusion of the second polar body;
c) culturing the exposed oocytes from step (b) *in vitro* under conditions resulting in formation of a blastocyst;
d) culturing inner cell mass cells of the blastocyst *in vitro* under conditions resulting in production of stem cells; and
e) screening the stem cells to identify stem cells that are homozygous for a recessive disease-causing allele.

The present invention also provides a method for producing differentiated cells which are homozygous for at least one recessive disease-causing allele, comprising:
a) activating unfertilized oocytes whose DNA contains a recessive disease-causing allele;
b) exposing the activated oocytes to chemical treatment that inhibits extrusion of the second polar body;
c) culturing the exposed oocytes from step (b) *in vitro* under conditions resulting in formation of a blastocyst;
d) culturing inner cell mass cells of the blastocyst *in vitro* under conditions resulting in production of stem cells;
e) screening the stem cells to identify stem cells that are homozygous for a recessive disease-causing allele; and
f) differentiation of the pluripotent stem cells.

The present invention further provides a method of identifying molecules that inhibit or block expression of a recessive disease-causing allele, comprising:
a) activating unfertilized oocytes whose DNA contains a recessive disease-causing allele;
b) exposing the activated oocytes to chemical treatment that inhibits extrusion of the second polar body;
c) culturing the exposed oocytes from step (b) *in vitro* under conditions resulting in formation of a blastocyst;
d) culturing inner cell mass cells of the blastocyst *in vitro* under conditions resulting in production of stem cells;
e) screening the stem cells to identify stem cells that are homozygous for a recessive disease-causing allele;
f) differentiation of the pluripotent stem cells; and
g) using the differentiated cells in a screening assay to identify molecules that inhibit or block the expression of said recessive disease-causing gene,
   wherein the screening assay is affected *in vitro.*

Further, the present invention provides a non-human animal model for study of a disease that is caused by the expression of a recessive disease causing gene which is produced by introducing into a non-human animal differentiated cells derived from parthenogenetically derived pluripotent stem cells obtained by the methods of the invention,
wherein the non-human animal is a murine animal model.

### BRIEF DESCRIPTION OF THE FIGURES

Figure 1 shows cynomulgous monkey blastocysts derived from parthenogenetic embryos.
Figure 2 shows an ES-like cell line (Cyno 1) derived from a cynomulgous parthenogenetic blastocyst.
Figure 3 and 4 show the Cyno 1 cell line before and after immunosurgery.
Figure 5 shows the Cyno 1 cell line 5 days after plating.
Figure 6 shows the Cyno 1 cell line growing on top of a feeder layer.
Figure 7 shows the results of an RT-PCR showing that the Cyno-1 cell line is homozygous for the Snrpn gene (contains paternal allele).
Figure 8 shows metaphase II oocytes at retrieval.
Figure 9 shows 4 and 6 cell embryo 48 hours after parthenogenetic activation.
Figure 10 shows blastocoele cavities in human parthenogenetic activation 48 hours after activation.
Figure 11 shows human parthenogenetic embryo having an inner cell mass.
Figure 12 shows human ES-like cells derived from cultured ICM cells.

### DESCRIPTION OF THE INVENTION

### A. A Bank Of Stem Cell Lines Homozygous For MHC Loci:

It is an object of the present description to prepare a bank of
pluripotent stem cell lines that are homozygous for one or more critical antigen genes, i.e., genes which encode histocompatibility antigens, e.g., in the case of human stem cells and "stem-like" cells, MHC alleles that are present in the human population. Preferably, this work will be homozygous for MHC alleles that are representative of at least most prevalent in the particular species, preferably human. Many of these lines will also have an ABO blood group type O-negative to make them broadly compatible across the different blood types. Stem cell lines of the present description can be induced to differentiate into cell types suitable for therapeutic transplant. Because the cells of the present description have homozygous MHC alleles, the chance of obtaining cells for transplant that have MHC alleles that match those of a patient in need of a transplant is significantly enhanced. Instead of having to find a six of six match between two sets of HLA-A, HLA-B, and HLA-DR antigens, a high level of histocompatibility is provided by the cells for transplant of the present description when either of the two HLA-A, HLA-B, and HLA-DR antigens of the prospective transplant recipient matches one of the corresponding homozygous HLA antigens of the cells for transplant. For example, a stem cell bank able to provide cells having an HLA-A/HLA-B match to a patient having any of the eight HLA-A and nine HLA-B antigens listed in Table 2 would require only 72 stem cell lines with homozygous HLA-A and HLA-B antigens; whereas a bank of stem cells with heterozygous HLA-A and HLA-B antigens would need to have 4032 different stem cell lines. To provide a library of heterozygous stem cell lines that match the WHO list of serological types would require obtaining stem cells having every combination of 28 different pairs of HLA-A antigens and 59 different pairs of HLA-B, to account for both the maternal and paternal alleles for each loci. The complexity of such a stem cell bank, i.e., the number of different cell lines required, would be 2,587,032. In contrast, a bank of stem cells homozygous for the same HLA-A and HLA-B antigens would only need to have a complexity of 1,652 stem cell lines to guarantee a match to a patient with HLA-A and -B antigens on the WHO list of serological types. The actual number required to meet the needs of a majority of patients will actually be less than this due to the non-random distribution of alleles in various populations around the world. Patients in need of bone marrow stem cell grafts who are homozygous in particular alleles are particularly sensitive to graft versus host disease when heterozygous bone marrow grafts are used. Stem cell grafts using stem cells having homozygous alleles made according to the methods of the present description would alleviate this common complication of transplants.

This present description provides novel means for making libraries of pluripotent stem cells that can serve as sources of cells for therapeutic transplant that are highly histocompatible with human or non-human patients in need of cell transplants. Additionally, those cell lines are useful in creating animal models for specific diseases that may be used to evaluate potential treatments and drug antidotes. In one aspect, the description comprises preparing a bank of stem cell lines that are homozygous for one or more critical antigen alleles, in the case of human stem cells. MHC alleles that are present in all or most of the world's populations, including the populations of North America, Central and South America, Europe, Africa, and Asia, and the Pacific islands. It is an object of the present description to provide a stem cell bank comprising stem cells generated from vertebrate somatic cells, preferably mammalian somatic cells, and more preferably human research cells that are homozygous for one or more critical antigen alleles, e.g., MHC alleles using nuclear transfer or parthenogenic produced embryos. A preferred object of the present description is to provide a stem cell bank comprising diploid vertebrate, preferably mammalian and more preferably human stem cells generated by parthenogenesis that are homozygous for MHC alleles.

The stem cell bank of the present description comprises lines of pluripotent stem cells that are homozygous for at least one histocompatibility antigen collection. In the case of human stem cells this will be an MHC allele selected from the group consisting of HLA-A, HLA-B, HLA-C, HLA-DR, HLA-DQ, and HLA-DP. In a useful aspect, the stem cell bank comprises pluripotent stem cells stem cells that are homozygous for the significant histocompatibility antigen alleles, e.g., the HLA-A, HLA-B, and HLA-DR alleles. In another aspect, the stem cell bank comprises stem cells that are homozygous for all of the histocompatibility antigen alleles, e.g., MHC alleles.

The stem cell bank of the present description can comprise parthenogenetically-derived embryonic stem (ES) cells, that can differentiate in vivo or ex vivo into a wide variety of different cell types having one or more homozygous MHC alleles. The stem cell bank of the present description can also comprise partially differentiated stem cells such as neuronal stem cells and/or hematopoietic stem cells, that differentiate in vivo or ex vivo into a more limited number of differentiated cell types having one or more homozygous MHC alleles. These stem cells optionally may be transgenic, e.g., they may express antigens that encode therapeutic or diagnostic proteins and polypeptides. For example, the stem cells may be genetically engineered to express proteins that inhibit immune rejection responses such as CD4O-L (CD154 or gp139) or in the case of porcine stem cells may be genetically engineered to knock-out a glycosylated antigen that is known to trigger immune rejection responses.

An object of the present description is to provide a stem cell bank comprising stem cells having homozygous histocompatibility alleles, i.e., MHC alleles that are available "off the shelf" for providing histocompatible cells suitable for transplant to patients in need of such a transplant. Desirably, this stem bank will include stem cell lines that are representative of the different histocompatibility antigens expressed in the particular species, e.g., human. In a useful aspect, the stem cell bank comprises stem cells that are isolated and maintained without feeder cells or serum of non-human animals, to minimize concerns of contamination by pathogens. In another useful aspect, the stem cell bank comprises stem cells that are genetically modified relative to the cells of the donor, e.g., human donor from which they are derived.

An object of the present description is to provide a stem cell bank comprising stem cells having homozygous recessive alleles responsible for genetically inherited diseases. Recessive disease-causing genes are endemic in the population, and such stem cells can be generated by parthenogenesis using oocytes collected from female carriers of the recessive disease-causing alleles. There is great need for
pluripotent stem cells having homozygous recessive disease-causing alleles that can be induced to differentiate into cells useful for basic research directed to studying the disease phenotype, both ex vivo and in vivo (e.g., in immunodeficient laboratory animals), and for screening to discover drugs and other therapies that treat or cure the disease.

### B. Terms Used in Herein:

As used herein, a "stem cell" is a cell that has the ability to proliferate in culture, producing some daughter cells that remain relatively undifferentiated, and other daughter cells that give rise to cells of one or more specialized cell types; and "differentiation" refers to a progressive, transforming process whereby a cell acquires the biochemical and morphological properties necessary to perform its specialized functions. Stem cells therefore reside immediately antecedent to the branch points of the developmental tree.

As used herein, an "embryonic stem cell line" is a cell line with the characteristics of the murine ES cells isolated from morulae or blastocyst inner cell masses, as reported by Martin (Proc. Natl. Acad. Sci. USA (1981) 78:7634-7638); and by Evans et al. (Nature (1981) 292: 154-156). ES cells have high nuclear-to-cytoplasm ratio, prominent nucleoli, are capable of proliferating indefinitely and can be differentiate into most or all of the specialized cell types of an organism, such as the three embryonic germ layers, all somatic cell lineages, and the germ line.

In some cases, ES cells are obtained that can differentiate into almost all of the specialized cell types of an organism; but not into one or a small number of specific cell types. For example, Thomson et al. describe isolating a primate ES cell that, when transferred into another blastocyst, does not contribute to the germ line (Proc. Natl. Acad. Sci. USA. (1995) 92:7844-7848).

As used herein, "inner cell mass-derived cells" (ICM-derived cells) are cells directly derived from isolated ICMs or morulae of parthenogenetically-derived embryos without passaging them to establish a continuous ES or ES-like cell line. Methods for making and using ICM-derived cells are described in co-owned U.S. Patent No. 6,235,970.

As used herein, "ex vivo" cell culture refers to culturing cells outside of the body. Ex vivo cell culture includes cell culture in vitro, e.g., in suspension, or in single- or multi-well plates. Ex vivo culture also includes co-culturing cells with two or more different cell types, and culturing in or on 2- or 3-dimensional supports or matrices, including methods for culturing cells alone or with other cell types to form artificial tissues.

As used herein, "parthenogenetic embryos" refers to an embryo that only contains male or female chromosomal DNA that is derived from male or female gametes. For example, parthenogenetic embryos can be derived by activation of unfertilized female gametes, e.g., unfertilized human, murine, cynomolgus or rabbit oocytes.

As used herein, the term "gene" refers to the nucleotide sequences at a genetic locus that encode and regulate expression of a functional mRNA molecule or a polypeptide; i.e., as used herein, a gene includes the nucleotide sequences that make up the coding sequence (exons and introns), the promoter, enhancers, and other DNA elements that regulate transcription, including as elements conferring cell type-specific and differentiation stage-specific expression, hormone responsive elements, repressor elements, etc., and nucleotide sequences that encode signals that regulate splicing and translation of the mRNA, such as a cleavage signal, a polyadenylation signal, or an internal ribosome entry site (IRES).

### C. Providing Histocompatible Transplants To Animal or Human Recipients:

Another object of the description is to provide a method by which a human or non-human animal, e.g., a person in need of a cell or tissue transplant can be provided with cells or tissue suitable for transplantation that have homozygous histocompatibility antigen alletes, e.g., in the case of human recipients MHC alleles that match the MHC alleles of the person needing the transplant. The description provides a method in which the MHC alleles of a person in need of a transplant (the recipient) are identified, and a line of stem cells homozygous for at least one MHC allele present in the recipient's cells is obtained from a stem cell bank produced according to the disclosed methods. That line of stem cells is then used to generate cells or tissue suitable for transplant that are homozygous for at least one MHC allele present in the recipient's cells. The cells or tissue so obtained can be grafted to the body of the person in need of such a transplant. In a useful aspect, three, four, five, six or more of the MHC alleles of the line of stem cells used to generate cells or tissue for transplant are homozygous and match MHC alleles of the transplant recipient.

In a useful aspect, the line of stem cells used to generate cells or tissue suitable for transplant is a line of hematopoietic stem cells. The lines of stem cells that can be used to generate cells or tissue suitable for transplant are available "off the shelf" in the stem cell bank.

In another useful aspect, the stem cell bank comprises one or more lines of pluripotent stem cell having DNA that is genetically modified relative to the DNA of the human donor from which they are derived. For example, the method of the description comprises altering genomic DNA of the cells to replace a non-homozygous MHC allele with one that is homozygous, or to inhibit the effective presentation of a class I or class II HLA antigen on the cell surface, e.g., by preventing expression of β2-microglobulin, or by preventing expression of one or more MHC alleles. Also, the method encompasses introducing one or more genetic modifications that result in lineage-defective stem cells, i.e., stem cells which cannot differentiate into specific cell lineages.

### D. Methods For Making Stem Cell Lines With Homozygous MHC Alleles:

Pluripotent stem cell lines that make up the stem cell banks of the present description can be derived from parthenogenetically-derived blastocyst embryos made up of cells that are homozygous for some or all of the histocompatibility antigen alleles, e.g., MHC alleles. Blastocyst embryos useful according to the present description can be made by parthenogenesis. In the case of human embryos, for ethical reasons, they are never allowed to develop beyond the stage of pre-implantation blastocysts of about 9-10 days before the inner cell mass cells are isolated and are cultured to produce embryonic stem (ES) cells.

### Stem cells produced by parthenogenesis:

According to the present invention the pluripotent stem cells are parthenogenetically-derived. In an embodiment of the invention, the pluripotent stem cells derived by parthenogenesis are pluripotent human stem cells. The stem cells obtained by this method are diploid, because extrusion of the second polar body following parthenogenetic activation is inhibited. Methods for producing a diploid human embryo by parthenogenesis, for culturing the embryo in vitro to form a blastocyst, and for culturing cells of the blastocyst to obtain stem cells, are described in co-owned and co-pending PCT Application PCT/USO2/37899 (Methods for Making and Using Reprogrammed Human Somatic Cell Nuclei and Autologous and Isogenic Stem Cells) filed November 26, 2002, and published as WO 03/046141 A2.

Similar methods for producing diploid embryos by parthenogenesis using oocytes of rhesus monkeys and cynomolgous monkeys have been described by Mitalipov et al. (2001, Biology of Reproduction, 65:253-259) and Cibelli et al. (2002, Science, 295:81), respectively.

In general, production of a diploid human embryo by parthenogenesis comprises
a. obtaining oocytes from human donors induced to superovulate by treatment with gonadotropins followed by hCG injection;
b. activating the oocytes at about 38-45 hours after hCG stimulation;
c. exposing the activated oocytes to chemical treatment that inhibits extrusion of the second polar body; and
d. culturing the embryo in vitro under conditions resulting in formation of a blastocyst.
Oocyte activation is normally mediated by oscillations of intracellular Ca⁺² ion triggered by the sperm cell. Parthenogenetic activation of the oocytes can be achieved by any of the known means for inducing oocyte activation. Such methods generally involve exposing the oocyte to ethanol, electroporation, calcium ionophore, ionomycin, or inositol 1,4,5-triphosphate to increase the intracellular Ca⁺² ion concentration in the oocyte, in combination with a treatment that temporarily inhibits protein synthesis or protein phosphorylation. For example, Mitalipov et al. (supra, p. 254) describe two such methods that result in production of diploid parthenogenetic blastocysts from oocytes of rhesus monkeys. In one method, the oocytes are incubated briefly in medium containing ionomycin and calcium, followed by incubation for several hours in medium containing 6-aminomethylpurine (DMAP), an inhibitor of protein phosphorylation. In the other method, the oocytes are electroporated three times in medium containing calcium, and between each electroporation, the oocytes are incubated for about 30 minutes in medium containing cycloheximide, an inhibitor of protein synthesis, and cytochalasin B, an inhibitor of microfilament synthesis.

Using a similar method Cibelli et al. (supra) parthenogenetically activated oocytes of a cynomolgous monkey; cultured the activated oocytes in vitro to produce a diploid blastocysts; and isolated a line of diploid ES cells from cells of the inner cell mass of a parthenogenesis-derived embryo; and showed that the ES cells are capable of differentiating into cell types of all three embryonic germ layers.

Oocytes having MHC alleles of the type needed for the stem cell bank are parthenogenetically activated and are cultured to form blastocysts. Using known methods, the inner cell mass cells of the blastocysts are cultured in vitro to generate diploid embryonic stem cells. Because extrusion of the second polar body after meiosis II was prevented, the homologous chromosomes of such ES cells are actually the sister chromatids that were joined together as a dyad during meiosis I. Since the sister chromatids were formed by replication of a single set of chromosomes at the outset of meiosis, they will have identical DNA sequences, except for those regions that were exchanged with the homologous dyad during the recombination stage of meiosis. The HLA genes of the MHC are tightly linked, and recombination in this region is rare. occurring with a frequency of about 1%. The two sets of homozygous HLA alleles in the parthenogenetically-derived stem cell lines obtained with oocytes from a given donor reflect the HLA haplotypes of the maternal and paternal copies of chromosome 6 that the donor inherited from her parents. Known screening methods can be performed to identify the cell lines that have non-homozygous HLA antigens due to genetic recombination, and to identify the homozygous HLA alleles of each stem cell line.

### Genetically modified stem cells:

The methods of the present description include producing pluripotent stem cells homozygous for MHC antigens that are genetically modified relative to the cells of the human donor from which they were originally obtained. The stem cells can be genetically modified in any manner that enhances or improves the overall efficiency by which cells for transplant are produced and the therapeutic efficacy of the cell transplantation. Methods that use recombinant DNA techniques to introduce modifications at selected sites in the genomic DNA of cultured cells are well known. Such methods can include (1) inserting a DNA sequence from another organism (human or non-human) into target nuclear DNA, (2) deleting one or more DNA sequences from target nuclear DNA, and (3) introducing one or more base mutations (e.g., site-directed mutations) into target nuclear DNA. Such methods are described, for example, in Molecular Cloning, a Laboratory Manual, 2nd Ed., 1989, Sambrook, Fritsch, and Maniatis, Cold Spring Harbor Laboratory Press; U.S. Pat. No. 5,633,067, "Method of Producing a Transgenic Bovine or Transgenic Bovine Embryo," DeBoer et al., issued May 27, 1997; U.S. Pat. No. 5,612,205, "Homologous Recombination in Mammalian Cells," Kay et al., issued Mar. 18, 1997; and PCT publication WO 93/22432, "Method for Identifying Transgenic Pre-Implantation Embryos,".

Such methods include techniques for transfecting cells with foreign DNA fragments and the proper design of the foreign DNA fragments such that they effect insertion, deletion, and/or mutation of the target DNA genome. For example, known methods for genetically altering cells that use homologous recombination can be used to insert, delete, or rearrange DNA sequences in the genome of a cell of the present description. A genetic system that uses homologous recombination to modify targeted DNA sequences in a mammalian cell to "knock-out" a cell's ability to express a selected gene is disclosed by Capecchi et al. in U.S. Patent Nos. 5,631,153 and 5,464,764.

Such known methods can be used to insert into the genomic DNA of a cell an additional (exogenous) DNA sequence comprising an expression construct containing a gene that is to be expressed in the modified cell. The gene to be expressed can be operably linked to any of a wide variety of different types of transcriptional regulatory sequences that regulate expression of the gene in the modified cell. For example, the gene can be under control of a promoter that is constitutively active in many different cell types, or one that is developmentally regulated and is only active in one or a few specific cell types. Alternatively, the gene can be operably linked to an inducible promoter that can be activated by exposure of the cell to a physical (e.g., cold, heat, light, radiation) or chemical signal. Many such inducible promoters and methods for using them effectively are well known. Examples of the characteristics and use of such promoters, and of other well-known transcriptional regulatory elements such as enhancers, insulators, and repressors, are described, for example, in Transgenic Animals, Generation and Use, 1997, edited by L. M. Houdebine, Hardwood Academic Publishers, Australia,

Stem cells homozygous for MHC antigens that have multiple genetic alterations can be produced using known methods. For example, one can produce cells that are modified at multiple loci, or cells that are modified at a single locus by complex genetic alterations requiring multiple manipulations. To produce stem cells having multiple genetic alterations, it is useful to perform the genetic manipulations on somatic cells cultured in vitro, and then to clone the genetically altered cells by somatic cell nuclear transfer and generate ES cells having multiple genetic alterations from the resulting blastocysts.

Alternatively, the pluripotent stem cells having homozygous MHC alleles that are produced by any of the methods described above can be genetically modified directly using known methods. For example, Zwaka et al. have described a method for genetically modifying human ES cells in vitro by homologous recombination (Nature Biotechnology, Vol. 21, No. 3, March, 2003).

Stem cells can be produced that are genetically modified grow more efficiently in tissue culture than unmodified cells; e.g., by increasing the number of growth factor receptors on the cells' surface. Use of stem cells having such modifications reduces the time required to generate an amount of cells for transplant that is sufficient to have therapeutic effect.

The histocompatibility of a line of cells to be used for transplant with a patient in need of such as transplant may be increased by altering the genomic DNA of the cells to replace a non-homozygous MHC allele with one that is homozygous and matches an HLA allele of the patient. Alternatively, the genomic DNA of the cells can be modified to inhibit the effective presentation of a class I or class II HLA antigen on the cell's surface; for example, by introducing a genetic alteration that prevents expression of *β*2-microglobulin, which is an essential component of class I HLA antigens; by introducing genetic alterations in the promoter regions of the class I and/or or class II MHC genes; or simply by deleting a portion of the DNA of one or more of the class I and/or or class II MHC genes sufficient to prevent expression of the gene(s).

Stem cells can be genetically modified (e.g., by homologous recombination) to have a heterozygous knock-out of the Id1 gene, and a homozygous knockout of the Id3 gene. As described In co-owned and co-pending PCT Application No. PCT/USU3/01827 (Stem Cell-Derived Endothelial Cells Modified to Disrupt Tumor Angiogenesis), filed January 22, 2003, and published as WO 03/061591 A2, these stem cells can be induced to differentiate into Id1+/-, Id3 -/- endothelial cell precursor cells that are useful for the treatment of cancer because they give rise to endothelial cells that disrupt and inhibit tumor angiogenesis.

Stem cells can also be genetically modified to provide a therapeutic gene product that the patient requires, e.g., due to an inborn error of metabolism. Many genetic diseases are known to result from an inability of a patient's cells to produce a specific gene product. Genetically altered stem cells can be made that can be used to produce cells with homozygous MHC alleles for transplantation that are genetically modified to synthesize enhanced amounts of a gene product required by the transplant recipient. For example, hematopoietic stem cells that are genetically altered to produce and secrete adenosine deaminase can be prepared for transplant to a patient suffering from adenosine deaminase deficiency. The methods of the present description permit production of such cells without the use of recombinant retrovirus, which can insert at a site in the genomic DNA that disrupts normal growth control and causes neoplastic transformation.

Stem cells of the description can also be genetically modified by introduction of a gene that causes the cell to die. The gene can be put under control of an inducible promoter. If for any reason the transplanted cells react in a in a way that can harm the recipient, expression of the suicide genes can be induced to kill the transplanted cells. Use of Inducible suicide genes in this manner is known in the art. Suitable suicide genes include genes encoding HSV thymidine kinase and cytodine deaminase, with which cell death is induced by gancyclovir and 5-fluorocytosine, respectively.

The cells may be modified to knockout one or more histocompatibility antigen alletes, e.g., MHC alleles such that only one set remains. This leads to an underexpression of the MHC genes, but a phenotype effective in reducing the complexity of the MHC serotype and effective in producing cells capable of otherwise functioning and useful in the treatment of disease. Alternatively, homozygosity can be engineered into the cell lines by the targeted introduction of the appropriate alleles to the nonhomologous set, to result in homozygosity. In addition, the chromosome carrying the MHC genes can be removed from cells by laser ablation and a chromosome carrying the identical chromosome as remains in the cell can be added by microsome-mediated chromosome transfer, or by other techniques known in the art.

The present description is by no means limited to the foregoing examples of genetic alterations. Persons skilled in the art will be able to identify numerous other ways by which stem cells produced according to the present description can be genetically modified to enhance their utility.

### Preparing pluripotent stem cells:

ES cell lines can be derived from parthenogenetically-derived human blastocysts using known methods comprising removing cells of the inner cell mass of an early blastocyst by microsurgery or immune-surgery and culturing the cells in vitro (e.g., see U.S. Patent No. 6,235,970). For example, such methods are described in co-owned and co-pending PCT application, PCT/US02/37899 (Methods for Making and Using Reprogrammed Human Somatic Cell Nuclei and Autologous and Isogenic Stem Cells) filed November 26, 2002, and published as WO 03/046141 A1, using blastocysts produced by parthenogenesis.

Thomson et al. also describes methods by which ES cell lines can be derived from rhesus monkey blastocysts (Proc. Natl. Acad. Sci., USA, 1995, 92:7544-7848).

Methods for growing human ES cells and maintaining them in an undifferentiated state without culturing them on a layer of feeder cells have also been described (Xu et al., Nature Biotechnology, 2001, 19:971-4). Feeder-free culture of stem cells can reduce the risk of contamination of the cells by pathogens that may reside in the feeder cells.

### Generating differentiated cells:

Stem cells are widely regarded as an abundant source of pluripotent cellular material that can be directed to differentiate into cells and tissues that are suitable for transplantation into patients in need of such cell and tissue transplants. ES cells appear to have unlimited proliferative potential and are capable of differentiating into all of the specialized cell types of a mammal, including the three embryonic germ layers (endoderm, mesoderm, and ectoderm), and all somatic cell lineages and the germ line. Using known methods, ES cells can be cultured under conditions in which they differentiate into multipotent stem cells such as hematopoietic or neuronal stem cells. Alternatively, ES cells can be cultured under conditions in which they differentiate into a terminally differentiated cell type such as a cardiac muscle cell. Pluripotent stem cells homozygous for MHC alleles produced by the methods of the present description can be cultured using methods and conditions known in the art to generate cell lineages that differentiate into many, if not all, of the cell types of the body, for transplant into human patients in need of such transplants. Such stem cells having one or more homozygous MHC alleles can differentiate into cells selected from the group consisting of immune cells, neurons, skeletal myoblasts, smooth muscle cells, cardiac muscle cells, skin cells, pancreatic islet cells, hematopoietic cells, kidney cells, and hepatocytes. For example, methods have been described by which ES cells are induced to differentiate in vitro into cardiomyocytes (Paquin et al., Proc. Nat. Acad. Sci. (2002) 99:9550-9555), hematopoietic cells (Weiss et al., Hematol. Oncol. Clin. N. Amer. (1997) 11(6):1185-98; also U.S. Patent No. 6,280,718), insulin-secreting beta cells (Assady et al., Diabetes (2001) 50(8):1691-1697), and neural progenitors capable of differentiating into astrocytes, oligodendrocytes, and mature neurons (Reubinoff et al., Nature Biotechnology (2001) 19:1134-1140; also U.S. Patent No. 5,851,832).

Novel screening methods that make use of gene trapped cell lines and provide means for efficiently identifying combinations of biological, biochemical, and physical agents or conditions that induce stem cells to differentiate into cell types useful for transplant therapy, and for preparing and isolating specific differentiated cell types, are described in co-owned and co-pending U.S. Application No. 10/227,282 filed August 26, 2002, and published as US 2003/0224345 A1.

In a useful aspect of the present description, a stem cell bank is produced that comprises hematopoietic stem cells homozygous for MHC antigens. A method for inducing the differentiation of pluripotent human embryonic stem cells into hematopoietic cells useful for transplant according to the present description is described in U.S. Patent No. 6,280,718, "Hematopoietic Differentiation of Human Pluripotent Embryonic Stem Cells," issued to Kaufman et al. on August 28, 2001.

The method disclosed in the patent of Kaufman et al. comprises exposing a culture of pluripotent human embryonic stem cells to mammalian hematopoietic stromal cells to induce differentiation of at least some of the stem cells to form hematopoietic cells that form hematopoietic cell colony forming units when placed in methylcellulose culture.

Those skilled in the art will appreciate that, using currently available methodologies, the pluripotent stem cells of the present description can also be used to generate tissues formed of two or more different cell types homozygous for a MHC allele, for transplant to a person in need of such a tissue transplant.

The pluripotent stem cells homozygous for MHC antigens that are generated according to the present description, and the lines of differentiated cells obtained from these stem cells, are produced and isolated under Good Manufacturing Practices (GMP) conditions.

### Providing Histocompatible Transplants To People Needing Them:

The methods for generating stem cells and differentiated cells having homozygous MHC alleles described above provide effective solutions to many of the problems associated with obtaining cells for transplant that are histocompatible with a transplant recipient. However, de novo production of histocompatible cells and tissue for transplantation parthenogenesis for each patient in need of transplant is time-consuming. The time required to prepare "customized" cells or tissue for transplantation having the same HLA antigens as the transplant recipient can be problematic when the health of the would-be recipient is rapidly deteriorating for want of a transplant. Therefore, one or more of the above-described methods for generating stem cells and differentiated cells having homozygous MHC alleles are used to produce a stem cell bank comprising many different lines of stem cells, each having a different combination of homozygous MHC alleles present in the population. When a patient is found to be in need of a particular type of cell transplant, a line of stem cells from the stem cell bank having homozygous MHC alleles matching those of the patient can be taken "off the shelf" and cultured under conditions causing them to differentiate into the type(s) of cells needed. The differentiated cells are then isolated using known methods, and are provided to the patient's physician for transplant.

Accordingly, the present description includes the process of identifying the type of cells needed for transplant, and the blood type and HLA antigens of the transplant recipient, selecting stem cells from the stem cell bank that differentiate into the cell type needed and have homozygous HLA antigens that match those of the transplant recipient; culturing the stem cells under conditions in which they differentiate into the cell type needed; isolating the differentiated cells needed for transplant; and providing these to the patient's physician for transplant into the patient.

The differentiated cells for transplant produced by these methods are homozygous for at least one HLA antigen present on cells of the transplant recipient. Histocompatibility of the cells for transplant and the recipient increases as a function of the number of homozygous HLA antigens of the cells for transplant that match HLA antigens of the recipient. The greater the number of homozygous HLA antigens of the cells for transplant that match HLA antigens of the recipient, the longer the graft is expected to survive without being rejected. The cells for transplant provided by the description will therefore have one, two, three, four, five, six, or more homozygous HLA antigens that match HLA antigens of the recipient. For example, cells for transplant produced according to the present description can have homozygous HLA-A, HLA-B, and HLA-DR antigens that match HLA antigens of the recipient. Alternatively, the cells for transplant produced according to the present description can have homozygous HLA-A, HLA-B, HLA-C, HLA-DR, HLA-DQ, and HLA-DP antigens that match HLA antigens of the recipient. The ability to select stem cells "off the shelf" to produce cells for transplant having a relatively high number of homozygous HLA antigens that match those of a prospective transplant recipient depends on the size and complexity of the stem cells bank. A stem cell bank containing from 100,000 to 200,000 different stem cell lines, each having a different combination of homozygous HLA-A, HLA-B, and HLA-DR antigens, is required in order to be able to provide cells with homozygous HLA-A, HLA-B, and HLA-DR antigens that match the corresponding HLA antigens of a large percentage of people in a diverse population such as that of North America. Use of cells from individuals with blood type O can avoid rejection based on ABO blood type; but there would have to be two versions of each cell type in the stem cell bank in order to provide matches to the Rh(+) and Rh(-) blood types. Accordingly, a stem cell bank containing several hundred thousand stem cell lines can be expected to provide "off the shelf" stem cells that can be used to generate differentiated cells needed for transplant that have homozygous HLA-A, HLA-B, and HLA-DR antigens matching those of a person in need of such a transplant.

The stem cell bank of the present description contains lines of pluripotent human stem cells, each having a specific combination of one or more homozygous HLA antigens. The lines of stem cells that can be used to generate cells or tissue suitable for transplant can be lines of human ES cells. The stem cell lines can also be pluripotent, partially differentiated stem cells such as myoblasts, hematopoietic stem cells, neuronal precursor cells, and endothelial cell precursor cells.

### Therapeutic cell transplantation:

Using the methods of the present description, a line of pluripotent stem cells can be selected from a bank of such stem cells that are homozygous for one or more histocompatibility antigen alleles, in the case of human stem cells, MHC alleles that match an MHC allele of a patient in need of transplant. For example, the stem cells can have homozygous HLA-A, HLA-B, and HLA-DR alleles that match HLA-A, HLA-B, and HLA-DR alleles of the patient. The stem cells are cultured ex vivo under conditions in which they are induced to differentiate into partially or fully differentiated cell types that are suitable for transplant and have homozygous MHC alleles that match MHC alleles of the patient in need of the transplant.
The partially or fully differentiated cells needed for transplant are isolated from other cell types, e.g., using antibody-based separation methods such as cell sorting or immunomagnetic beads, and antibodies that are specific for one or more differentiation antigens on the surface of the cell type needed for transplant.

The isolated partially or fully differentiated cells are then administered by transplantation to the patient using known methods. Methods for transplantation of epidermal cells, hematopoietic stem cells, Islet of Langerhans cells, chondrocytes, hepatocytes, myoblasts, neural cells, and endothelial cells are reviewed by Inverardi et al. (Transplantation Biology, Cellular and Molecular Aspects, Chapter 56, 1996, ed. by Tilney et al., Lippincott-Raven, Philadelphia, PA). The method to be used to transplant or engraft cells to a patient is recognized as depending on the type of cells to be transplanted, and on the pathology of the patient.

### Cells Homozygous for Recessive Disease-Causing Genes:

Recessive alleles responsible for genetically inherited diseases are endemic in the population. If cells of people carrying a recessive disease-causing gene are used to produce stem cells having homozygous HLA alleles from embryos generated by parthenogenesis,
there is a relatively high likelihood that some of the stem cell lines obtained also be homozygous for the recessive disease-causing gene. The stem cell lines produced by the methods of the present invention can therefore be screened to identify those which are homozygous for a recessive disease-causing gene. Such screening can be carried out using known methods. For example, DNA sequences of the cells can be amplified by the polymerase chain reaction (PCR) and analyzed by DNA sequencing, restriction enzyme cleavage, or by hybridization to an array of oligomers, e.g., on a microchip. Examples of recessive-disease causing genes to be screened for include, but are not limited to, of recessive genes causing the following conditions:
Adenosine deaminase deficiency
Albinism
Adenylosuccinate lyase deficiency
Alpha-1 antitrypsin deficiency
Cystic Fibrosis
Friedreich's ataxia
Gaucher's disease
hypercholesterolemia
Alzheimer's Disease
Autoimmune polyendocrinopathy candidiasis-ectodermal dystrophy
AID - deficiency of activation-induced cytidine deaminase
Ataxia-telangiectasia
CD3-epsilon deficiency (causes SCID)
CD3-gamma deficiency (causes SCID)
chronic granulomatous disease - deficiency of p47^{phox}
Phenylketonuria - Phenylalanine hydroxylase (PAH) deficiency
Tetrahydrobiopterin deficiencies:
   GTP cyclohydrolase I (GTPCH) deficiency
   6-Pyruvoyl-tetrahydropterin synthase (PTPS) deficiency
   Dihydropteridine reductase (DHPR) deficiency
   Pterin-4a-carbinolamine dehydratase (PCD) deficiency
Janus Kinase 3 (JAK3) deficiency (causes SCID)
Hereditary fructose intolerance
Porphyria (one of the six forms is caused by a recessive gene)
Sickle Cell Anemia
Tay Sachs syndrome
Thalassemia
Wilson's disease
Xeroderma pigmentosum
Zeta-chain-associated protein kinase deficiency (causes SCID)

The pluripotent stem cell lines having a homozygous recessive disease-causing gene that are produced by the methods of the present invention are highly useful. They can be cultured under conditions in which they differentiate into cell types related to manifestation of the disease phenotype. Such cells having a homozygous recessive disease-causing gene are useful for basic research directed to studying the disease phenotype ex vivo. They can also be implanted into experimental animals (e.g., immunodeficient animals), for study of their metabolic activities in vivo. According to the invention such non-human animal models are murine animal models. Persons skilled in the art would recognize that studies in which such cells are genetically modified can be useful for gaining understanding of the disease phenotype. Such cells having a homozygous recessive disease-causing gene can also be used in drug discovery; e.g., in screening for drugs or other therapies that will treat or cure the disease caused by the recessive gene.

In order to further illustrate the invention and its preferred embodiments, the following examples are provided. These examples are intended to be exemplary and in no way limitative of the scope of the present invention, which is defined by the claims.

### EXAMPLE 1

### Production of Parthogenic Primate Primordial Stem Cells (PPSC's)

### Materials and Methods

1. Cynomolgous Monkey (Macaca fascicularis) were superovulated using a single injection of 1000 IU of pregnant mare's serum gonadrophin (PMSG) and 500 IU of human chorionic gonadoprophin (hCG) four days later.
2. Ovaries were retrieved by laparotomy and oocytes dissected from the follicles and placed in maturation media 36 to 48 hrs after (hCG). Maturation media consisted of medium-199 (Gibco BRL) with Earle's balanced salt solution supplemented with 20% fetal bovine serum, 10 IU/ml of PMSG, 10 IU/ml of hCG, 0.05 mg/ml of penicillin G and 0.075 mg/ml of steptomycin sulfate (Hong, 1999).
3. Oocyte activation
   After 40 hrs in maturation, metaphase II eggs were placed in 10 micromoles of lonomycin followed incubation in 200 mM 6-DMAP (dimethylaminopurine) for 3 to 4 hrs.
4. Embryo culture. Commercially available embryo culture media 'Cooks' was used (modified SOF). Embryos were cultured with a co-culture of mitotically inactivated mouse embryonic fibroblasts as feeder layer.
5. Isolation of inner cell mass
   a) Upon development to blastocyst, embryos were placed in a buffered solution of 0.3% pronase for 2 minutes to digest zona pellucida
   b) Blastocyts were then rinsed in buffered solution and moved to solution of G1 culture media and polyclonal antibodies (antihuman whole serum) 1:3 dilution for 30 minutes.
   c) Embryos were rinsed 5 times in a buffered solution.
   d) Embryos were moved into a solution of G1 culture media and guinea pig complement 1:3 dilution for 30 minutes.
   e) Remaining of the embryos (dead trophoblast cells and ICM) wee rinsed 5 times in buffered solution the Inner Cell Mass (ICM) was isolated and placed on top of a mouse embryonic fibroblast feeder layer for isolation and growth of Primordial Stem Cells (PSC's).

### Results

We have obtained 450 eggs total, after maturation, 224 were still at germinal vesicle stage (GV = no maturation), 79 were dead, 56 were at metaphase one (MI) and 91 at metaphase two (MII).

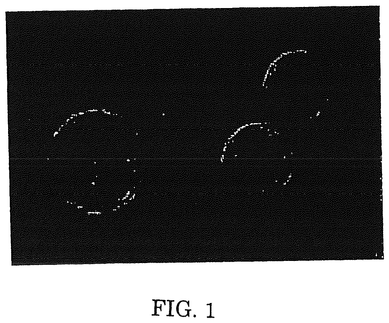

We have parthenogenically activated all of them. As expected, there was no cleavage on the GV group, 32% cleavage on the MI and 57% on the MII. When put in culture, 7 embryos developed to the blastocyst stage (See Figure 1).

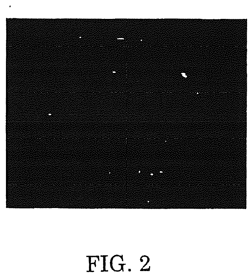

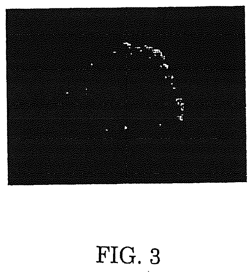

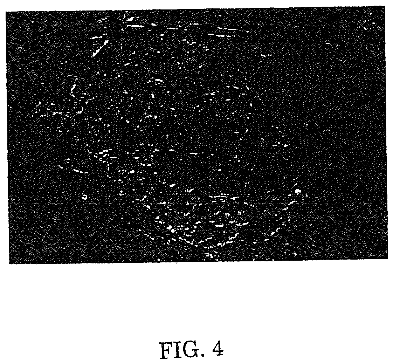

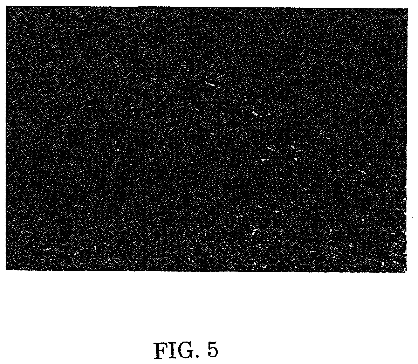

After attempting to establish ES-like culture cells, four Inner cell masses attached nicely one differentiated immediately, and out of the three remaining, one cell line was obtained. This cell line is called Cyno 1 (Fig. 2). This cell line before and after immunosurgery is shown in Figures 3 and 4. Figure 5 shows the Cyno 1 Cell line five days after plating.

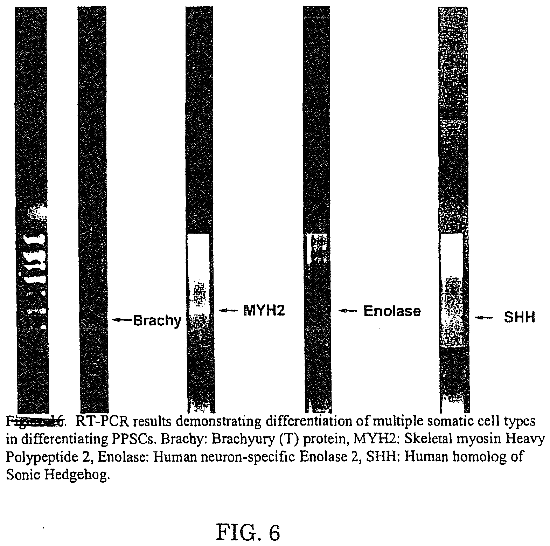

Figure 6 shows the Cyno 1 cell line growing on top of a mouse fibroblast feeder layer. These cells show typical morphology of pluripotent-embryonic-cells such as small nuclear cytoplasmic ration and the presence of cytoplasmic granules.

These cells were maintained in an undifferentiated state for a period of months. This is evidenced by screening of such cells after prolonged culturing for the expression of a cell marker characteristic of undifferentiated cells, Alkaline Phosphatase. As expected, cells were positive on passage 3 and on passage 5.

The fact that these cells maintain their pluripotency is also shown by their spontaneous differentiation into many differentiated cell types after being placed in tissue culture in the absence of a feeder layer. In the days following, the cells were observed to differentiate into cuboidal epithelium, fibroblasts, beating myocardial cells and other cells. Two colonies of beating myocardial cells wee observed in one well of a 4-well tissue culture plate.

To determine whether differentiated cells of various somatic cell lineages were observed from the differentiating PPSC's, we extracted mRNA from differentiated cell cultures, performed RT-PCR, using human sequence primers specific for various differentiated cell types. As shown in figure 6, transcripts of a predicted size for the mesodermally-derived transcripts brachyury and skeletal muscle myosin heavy polypeptide 2 were observed. The transcript sonic hedgehog essential for endoderm development was observed. In addition, the neuron-specific ectoderm marker enolase was observed as well as keratin (not shown) as markers of ectodermally derived cells. These PCR products were not observed in the mouse feeder layer controls or in the absence of reverse transcriptase.

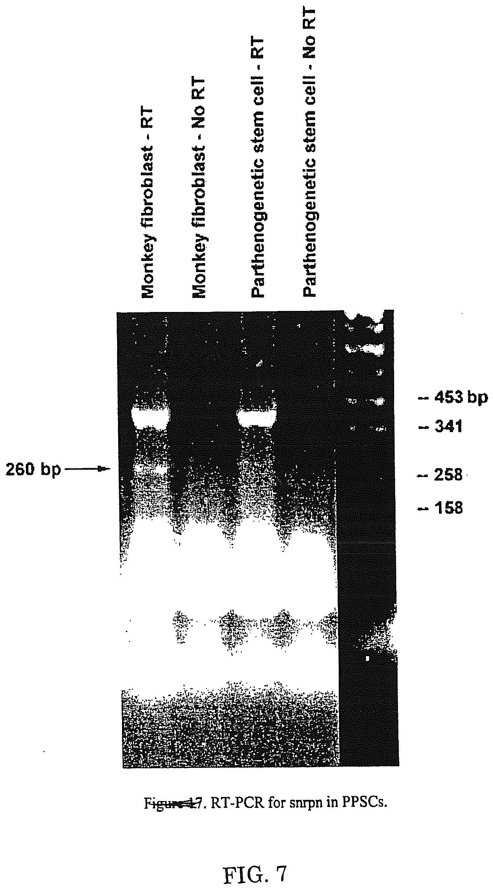

To establish that the imprinting status of parthogenetic PPSC's different than that of di-parental PPSCs we looked at the expression of several imprinted genes. Genes that are mono-allelically expressed from the paternal allele, would not be expected to be expressed in parthogenetic cells, as these cells are derived exclusively from the maternal genome. The *Snrpn* gene is mono-allelically expressed from the paternal allele in mouse blastocyst inner cell mass [Szabo,PE and Mann, JR; Genes & Development 9:3097-3108 (1995)]. We looked at the expression of this gene in the parthogenetic *Macaca facicularis* PPSCs and found that the express was undetectable by RT-PCR, whereas under identical conditions, this gene is readily detected in fibroblast cell cultures from the same species. The *Snrpn* gene is expected to be expressed in diparental PPSCs, as these cells contain a paternal allele. In Figure 7, the expected size RT-PCR product for the *Snrpn* gene is 260 bp.

### EXAMPLE 2

### Stable Engraftment of Homozygous Fibroblasts in Histocompatible and Non-Histocompatible Cynomolgous Recipients

Connective tissue fibroblasts are generated from the cyno-1 stem cell line described above which are labeled with green flourescent protein (GFP) gene. This cell line is homozygous as evidenced by the portion of a single allete of 225 basepairs using a primer set specific for DQBlu6011-17. These cells are propogated in vitro until several million cells are obtained.

Thereafter, approximately a million labeled connective fibroblasts are transplanted into histocompatible cynomolyus monkey recipients, and non-histocompatible cynomolgous controls. Each monkey is transplanted with a million labeled cells administered by injection in the upper arm at for different sites, in four equal parts.

The degree of engraftment of these engrafted labeled cells is assessed at three different times, at four weeks, six months and a year. Three of the four grafts are removed at three different times and the number of GFP labeled cells is determined in the histocompatible transplant recipients and controls. The number of GFP cells is compared for both groups.

Also, a histological examination is effected to look for any signs of lymphocyte infiltration and any signs of rejection.

### EXAMPLE 3

### Production of Homozygous Stem Cell Lines from Rabit Parthenogenically Activated Ooyctes

Rabbit ES cells were similarly obtained from parthenogenetic embryos. Specifically, rabbit oocytes were obtained from superovulating rabbits and were acttuated using ionomycin and DMAP. This resulted in blastocystes, the inner cell masses of which were transferred to fibroblast factor layer. This in turn resulted in the production of rabbit ES cell lines which stained positive for characteristic embryonic antigens and which gave rise to various differentiated cell types when removed from the front layer.

More specifically, true rabbit ES cell lines morphlogically looked like ES cells and differentiated into into all three germ cell linenages. Among the cell types that observed from this cell line were myocordial, vascular endothalial, neuronal, and hemotopoiath cell lineages.

### EXAMPLE 4

### Protection of Homozygous Stem Cell Lines from Human Parthenogenically Activated Oocytes

### Production of Autologous Cells by Parthenogenetic Activation of Oocytes

Oocytes from three volunteers were used for parthenogenetic activation. The donors were induced to superovulate by 11 days of low dose (75 IU bid) gonadotropin injections prior to hCG injection. A total of 22 oocytes were obtained from the donors 34 hours after HCG stimulation, and were activated at 40-43 h after hCG stimulation.

The oocytes were activated on day 0, using the ionomycin/DMAP activation protocol described above. Twelve hours after activation, 20 oocytes (90%) developed one pronucleus and cleaved to the two-cell to four-cell stage on day 2. On day 5 of culture, evident blastocoele cavities were observed in six of the parthenotes (30% of the cleaved oocytes) though none of the embryos displayed a clearly discemible inner cell mass. The results of parthenogenetic activation of the human oocytes are summarized in Table 4.

**Table 4, Parthenogenetic Activation of Human Oocytes**

| Donor | No. of Oocytes | Pronucleus (%)^{a} | Cleaved (%)^{a} | Embryos with blastocoele Cavity (%)⁵ |
|---|---|---|---|---|
| 1 | 5 | 4(80) | 4(80) | 0 |
| 2 | 14 | 13(93) | 13(93) | 4(31) |
| 6 | 5 | 3 | 3(100) | 2(67) |
| Total | 22 | 20(90) | 20(90) | 6(30) |

| | | | | |
|---|---|---|---|---|
| ^{a} As a percentage of activated oocytes. ^{b} As percentage of cleaved oocytes. | | | | |

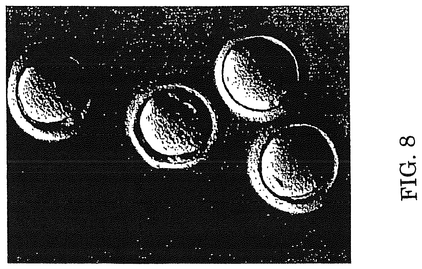

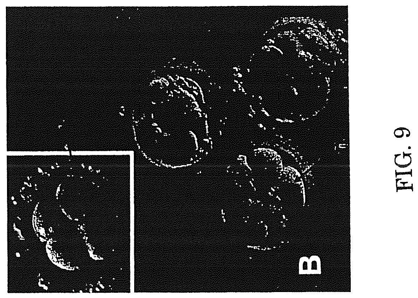

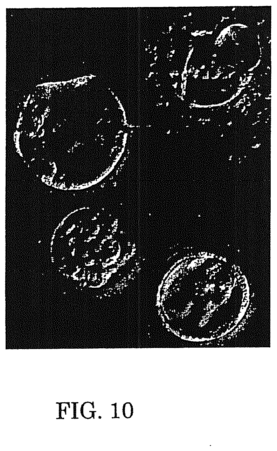

Figure 8 shows MII oocytes at the time of retrieval. Figure 9 shows four-to six-cell embryos 48 h after activation. Distinguishable single-nucleated blastomeres (labeled "n" in Fig. 6) were consistently observed. Figure 10 shows embryos with blastocoele cavities (arrows) that were detected on day 6 and maintained in culture until day 7. The scale bars for Figures 6 - 8 = 100 µm.

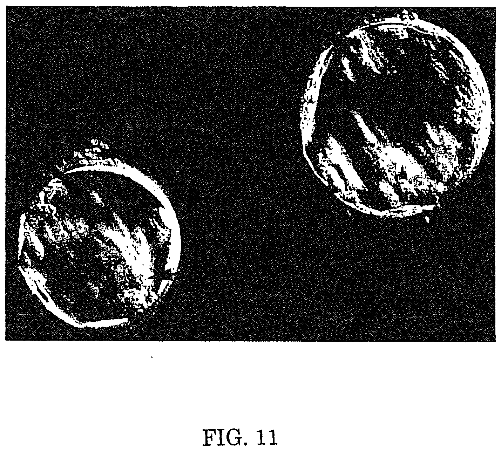

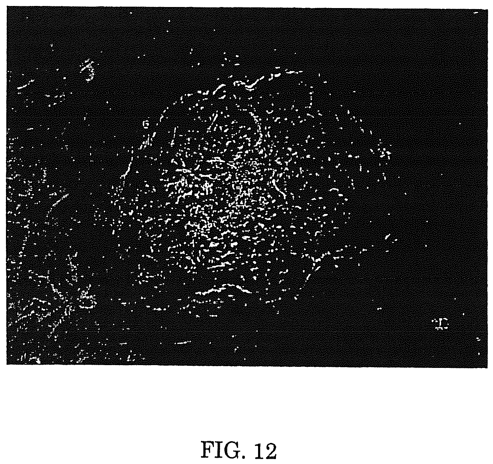

In a study similar to the one described above, human oocytes were activated using the ionomycin/DMAP activation protocol and were cultured *in vitro.* One of the activated embryos developed a pronucleus, cleaved, formed a blastocoele cavity, and then developed into a blastocyst having an inner cell mass, shown in Figure 11. The inner cell mass was isolated and plated on mouse feeder layers as described (Cibelli, J.B., et al. 2002. Parthenogenetic stem calls in non-human primates. Science 295: 819). The cultured ICM cells increased in number over the first week, and cells indistinguishable from human embryonic stem cells were observed. These grew in close association as a colony with a distinct boundary, as shown in Figure 12; they had a high nuclear-to-cytoplasmic ratio, prominent nucleoli, and were observed to differentiate *in vitro* into multiple differentiated cell types.

### EXAMPLE 5

### Production of Homozygous Stem Cell Lines from Mouse Parthenogenically Activated Oocytes

Using substantially the same methods described in the present application, another research group, Lin et al., Stem Cells 21:152-161 (2003), generated stem cell lines from unfertilized mouse metaphase II oocytes. These oocytes were activated by 5 minute exposure to 5mm calcium ionophore (ionomycin) followed by a 3 hour exposure to 6-methyldiaminopurine (DMAP). Those stem cell lines were characterized as stem cell lines based on their expression of characteristic embryonic antigens (SSEAs, OCT-4, alkaline phosphatase and telomerase) and their pluripotency (give rise to ectodermal, endodermal and mesodermal cell types).

Specifically, activated, unertilized oocytes from F1 hybrid mice (H-2-B/D0 were used to establish those stem cell lines homozygous for H-2-B and H-2-D respectively. The stem cell lines appeared karyotypically normal. When cultured in vitro in the pressures of specific growth factors, these cell lines gave rise to ectodermal, mesodermal, and enodermal cell types. Histological examination of cultures revealed cells having the morphology of neuronal cells and hemotopviette lineages (lymphocytes, monocytes and erythrocytes).

Further, when these cell lines were implanted in the kidney of syngenetic F1 mice they similarly resulted in teratomas that comprised cells of all three germ layers. The teratemas when histologically examined showed evidence of hair follicles, thyroid glands, lung epithelium and connective tissue.

### CONCLUSIONS

The results in the foregoing examples provides proof of principle, namely that homozygous stem cell lines may be generated from parthenogenically activated embryos, and used to produce differentiated cell types for cell therapy. More specifically, the present instruction provides methods for making libraries or banks of stem cell lines that are homozygous for specific MHC alleles. Thereby, a bank of cells is available which can be used to produce differentiated cells which are histocompatible for a wide range of transplant recipients. This is feasible with a relatively few number of stem cell lines given that certain HLA haplotyes are expressed with relatively high frequency in the human population.

These differentiated cells should be well tolerated and be stably engrafted given their antigenic expression relative to the transplant recipient. Also, stem cell lines derived from parthenogenically activated oocytes eliminate certain ethical issues with therapeutic cloning, namely a viable embryo (capable of giving rise to an offspring) is never obtained or destroyed. These cells are useful for treating any condition wherein cell or tissue transplantation is therapeutically desirable, e.g. immune deficiencies, age-related deficiencies, cancer, autoimmune disorder, organ deficiencies, disease, or injury, burn, malignancy, cell proliferation disorders, hemotopoietic disorders, e.g. blood malignancy such as non-Hodgkins lymphoma, leukemia, inflamatory disorders, connective tissue disorder, dermatological disorder, ischemia, stroke, neurological disorders and the like. The present cell banks on particularly well suited for treating acute disease, particularly when there is not sufficient time to do therapeutic cloning. For example, those cells are useful in obtaining differentiated cells for treatment of conditions where the patient is near death, e.g., sepsis, stroke and other conditions where cell therapy is urgently needed. Also, the description provides means for having cells on hand that express desired therapeutic polypeptides which are histocompatible.

## Claims

1. A pluripotent stem cell line homozygous for at least one recessive disease-causing allele that is parthenogenetically-derived produced by the method comprising:
a) activating unfertilized oocytes whose DNA contains a recessive disease-causing allele;
b) exposing the activated oocytes to chemical treatment that inhibits extrusion of the second polar body;
c) culturing the exposed oocytes from step (b) *in vitro* under conditions resulting in formation of a blastocyst;
d) culturing inner cell mass cells of the blastocyst *in vitro* under conditions resulting in production of stem cells; and
e) screening the stem cells to identify stem cells that are homozygous for a recessive disease-causing allele, thereby obtaining a
homozygous pluripotent stem cell for at least one recessive disease-causing allele.

2. The pluripotent stem cell line of claim 1, wherein the cells are human or murine.

3. The pluripotent stem cell line of claim 1, wherein the at least one recessive disease-causing allele causes a pathological condition selected from the group consisting of Adenosine deaminase deficiency, Albinism, Adenylosuccinate lyase deficiency, Alpha-1 antitrypsin deficiency, Cystic Fibrosis, Friedreich's ataxia, Gaucher's disease, hypercholesterolemia, Alzheimer's Disease, Autoimmune polyendocrinopathy candidiasis- ectodermal dystrophy, deficiency of activation-induced cytidine deaminase, Ataxia- telangiectasia, severe combined immunodeficieny (SCID), chronic granulomatous disease, Phenylketonuria, Tetrahydrobiopterin deficiency, Hereditary fructose intolerance, Porphyria (one of the six forms is caused by a recessive gene), Sickle Cell Anemia, Tay Sachs syndrome, Thalassemia, Wilson's disease, and Xeroderma pigmentosum.

4. A method for producing pluripotent stem cells homozygous for at least one recessive disease-causing allele, comprising:
a) activating unfertilized oocytes whose DNA contains a recessive disease-causing allele;
b) exposing the activated oocytes to chemical treatment that inhibits extrusion of the second polar body;
c) culturing the exposed oocytes from step (b) *in vitro* under conditions resulting in formation of a blastocyst;
d) culturing inner cell mass cells of the blastocyst *in vitro* under conditions resulting in production of stem cells; and
e) screening the stem cells to identify stem cells that are homozygous for a recessive disease-causing allele.

5. A method for producing differentiated cells which are homozygous for at least one recessive disease-causing allele, comprising:
a) activating unfertilized oocytes whose DNA contains a recessive disease-causing allele;
b) exposing the activated oocytes to chemical treatment that inhibits extrusion of the second polar body;
c) culturing the exposed oocytes from step (b) *in vitro* under conditions resulting in formation of a blastocyst;
d) culturing inner cell mass cells of the blastocyst *in vitro* under conditions resulting in production of stem cells;
e) screening the stem cells to identify stem cells that are homozygous for a recessive disease-causing allele; and
f) differentiation of the pluripotent stem cells.

6. A method of identifying molecules that inhibit or block the expression of a recessive disease-causing gene comprising:
a) activating unfertilized oocytes whose DNA contains a recessive disease-causing allele;
b) exposing the activated oocytes to chemical treatment that inhibits extrusion of the second polar body;
c) culturing the exposed oocytes from step (b) *in vitro* under conditions resulting in formation of a blastocyst;
d) culturing inner cell mass cells of the blastocyst *in vitro* under conditions resulting in production of stem cells;
e) screening the stem cells to identify stem cells that are homozygous for a recessive disease-causing allele;
f) differentiation of the pluripotent stem cells; and
g) using the differentiated cells in a screening assay to identify molecules that inhibit or block the expression of said recessive disease-causing gene,
wherein the screening assay is affected in vitro.

7. A non-human animal model for study of a disease that is caused by the expression of a recessive disease causing gene which is produced by introducing into a non-human animal differentiated cells from parthenogenetically derived pluripotent stem cells obtained according to the method of claim 5, wherein the non-human animal model is a murine animal model.

## Patentansprüche

1. Pluripotente Stammzelllinie, das homozygot für wenigstens ein rezessives krankheitserregendes Allel ist, das parthenogenetisch abgeleitet ist, erzeugt durch das Verfahren umfassend:
a) Aktivieren unbefruchteter Oozyte, deren DNA ein rezessives krankheitserregendes Allel beinhaltet;
b) Aussetzen der aktivierten Oozyte einer chemischen Behandlung, die das Extrudieren des zweiten Polkörpers hemmt;
c) Kultivieren der ausgesetzten Oozyten von Schritt (b) *in vitro* unter Bedingungen, die zu einer Ausbildung einer Blastozyste führen;
d) Kultivieren von Zellen der inneren Zellmasse der Blastozyste *in vitro* unter Bedingungen, die zu einer Erzeugung von Stammzellen führen; und
e) Screenen der Stammzellen, um Stammzellen zu identifizieren, die homozygot für ein rezessives krankheitserregendes Allel sind, wobei dadurch eine homozygote pluripotente Stammzelle für wenigstens ein rezessives krankheitserregendes Allel erhalten wird.

2. Pluripotente Stammzelllinie nach Anspruch 1, wobei die Zellen menschlich oder murin sind.

3. Pluripotente Stammzelllinie nach Anspruch 1, wobei das wenigstens eine rezessive krankheitserregende Allel einen pathologischen Zustand verursacht, der aus der Gruppe ausgewählt ist, die aus Folgendem besteht: Adenosindesaminase[ADA]-Mangel, Albinismus, Adenylsuccinat-Lyase-Mangel, Alpha-1-Antitrypsinmangel, zystischer Fibrose, Friedreich-Ataxie, Gaucher-Krankheit, Hypercholesterinämie, Alzheimer-Krankheit, autoimmuner polyglandulärer Insuffizienz, Immundefekt bei erhöhtem Immunglobulin M, Ataxia teleangiectatica, schwerem kombiniertem Immundefekt *[severe combined immunodeficieny-*SCID], chronischer Granulomatose, Phenylketonurie, Tetrahydrobiopterinmangel, hereditärer Fruktoseintoleranz, Porphyrie (eine der sechs Formen wird durch ein rezessives Gen verursacht), Sichelzellenanämie, Tay-Sachs-Krankheit, Thalassämie, Wilson-Krankheit und Xeroderma pigmentosum.

4. Verfahren zum Erzeugen pluripotenter Stammzellen, die homozygot für wenigstens ein rezessives krankheitserregendes Allel sind, umfassend:
a) Aktivieren unbefruchteter Oozyte, deren DNA ein rezessives krankheitserregendes Allel beinhaltet;
b) Aussetzen der aktivierten Oozyte einer chemischen Behandlung, die das Extrudieren des zweiten Polkörpers hemmt;
c) Kultivieren der ausgesetzten Oozyten von Schritt (b) *in vitro* unter Bedingungen, die zu der Ausbildung einer Blastozyste führen;
d) Kultivieren von Zellen der inneren Zellmasse der Blastozyste *in vitro* unter Bedingungen, die zu der Erzeugung von Stammzellen führen; und
e) Screenen der Stammzellen, um Stammzellen zu identifizieren, die homozygot für ein rezessives krankheitserregendes Allel sind.

5. Verfahren zum Erzeugen von differenzierten Zellen, die homozygot für wenigstens ein rezessives krankheitserregendes Allel sind, umfassend:
a) Aktivieren unbefruchteter Oozyte, deren DNA ein rezessives krankheitserregendes Allel beinhaltet;
b) Aussetzen der aktivierten Oozyte einer chemischen Behandlung, die das Extrudieren des zweiten Polkörpers hemmt;
c) Kultivieren der ausgesetzten Oozyten von Schritt (b) *in vitro* unter Bedingungen, die zu der Ausbildung einer Blastozyste führen;
d) Kultivieren von Zellen der inneren Zellmasse der Blastozyste *in vitro* unter Bedingungen, die zu der Erzeugung von Stammzellen führen;
e) Screenen der Stammzellen, um Stammzellen zu identifizieren, die homozygot für ein rezessives krankheitserregendes Allel sind; und
f) Differenzieren der pluripotenten Stammzellen.

6. Verfahren zum Identifizieren von Molekülen, die die Expression eines rezessiven krankheitserregenden Gens hemmen oder blockieren, umfassend:
a) Aktivieren unbefruchteter Oozyte, deren DNA ein rezessives krankheitserregendes Allel beinhaltet;
b) Aussetzen der aktivierten Oozyte einer chemischen Behandlung, die das Extrudieren des zweiten Polkörpers hemmt;
c) Kultivieren der ausgesetzten Oozyten von Schritt (b) *in vitro* unter Bedingungen, die zu der Ausbildung einer Blastozyste führen;
d) Kultivieren von Zellen der inneren Zellmasse der Blastozyste *in vitro* unter Bedingungen, die zu der Erzeugung von Stammzellen führen;
e) Screenen der Stammzellen, um Stammzellen zu identifizieren, die homozygot für ein rezessives krankheitserregendes Allel sind;
f) Differenzieren der pluripotenten Stammzellen; und
g) Verwenden der differenzierten Zellen in einem Screeningtest, um Moleküle zu identifizieren, die die Expression des rezessiven krankheitserregenden Gens hemmen oder blockieren, wobei der Screeningtest in vitro beeinflusst wird.

7. Nichtmenschliches Tiermodell zur Untersuchung einer Krankheit, die durch die Expression eines rezessiv krankheitserregenden Gens verursacht wird, das durch Einführen differenzierter Zellen aus parthenogenetisch abgeleiteten pluripotenten Stammzellen, die nach dem Verfahren nach Anspruch 5 erhalten wurden, in ein nichtmenschliches Tier erzeugt wird, wobei das nichtmenschliche Tiermodell ein murines Tiermodell ist.

## Revendications

1. Lignée de cellules souches pluripotentes homozygotes pour au moins un allèle pathogène récessif, dérivée par parthénogénèse et élaborée suivant le procédé consistant à :
a) activer des ovocytes non fertilisés dont l'ADN contient un allèle pathogène récessif ;
b) exposer les ovocytes activés à un traitement chimique qui inhibe l'extrusion du second globule polaire ;
c) cultiver les ovocytes exposés lors de l'étape (b) *in vitro* dans des conditions conduisant à la formation d'un blastocyste ;
d) cultiver des cellules de l'embryoblaste du blastocyste *in vitro* dans des conditions conduisant à l'élaboration de cellules souches ; et
e) cribler les cellules souches pour identifier des cellules souches homozygotes pour un allèle pathogène récessif, obtenant ainsi une cellule souche pluripotente homozygote pour au moins un allèle pathogène récessif.

2. Lignée de cellules souches pluripotentes selon la revendication 1, dans laquelle les cellules sont humaines ou murines.

3. Lignée de cellules souches pluripotentes selon la revendication 1, dans laquelle l'au moins un allèle pathogène récessif provoque un état pathologique choisi dans le groupe comprenant le déficit en adénosine-désaminase, l'albinisme, le déficit en adénylosuccinate lyase, le déficit en alpha-1 antitrypsine, la fibrose kystique, l'ataxie de Friedreich, la maladie de Gaucher, l'hypercholestérolémie, la maladie d'Alzheimer, la polyendocrinopathie-candidose-dystrophie ectodermale autoimmune, le déficit en cytidine désaminase induite par activation, l'ataxie-télangiectasie, un déficit immunitaire combiné sévère (DICS), la granulomatose chronique, la phénylcétonurie, un déficit en tétrahydrobioptérine, la fructosémie héréditaire, la porphyrie (dont l'une des six formes est provoquée par un gène récessif), la drépanocytose, la maladie de Tay-Sachs, la thalassémie, la maladie de Wilson et le xeroderma pigmentosum.

4. Procédé d'élaboration de cellules souches pluripotentes homozygotes pour au moins un allèle pathogène récessif, consistant à :
a) activer des ovocytes non fertilisés dont l'ADN contient un allèle pathogène récessif ;
b) exposer les ovocytes activés à un traitement chimique qui inhibe l'extrusion du second globule polaire ;
c) cultiver les ovocytes exposés lors de l'étape (b) *in vitro* dans des conditions conduisant à la formation d'un blastocyste ;
d) cultiver des cellules de l'embryoblaste du blastocyste *in vitro* dans des conditions conduisant à l'élaboration de cellules souches ; et
e) cribler les cellules souches pour identifier des cellules souches homozygotes pour un allèle pathogène récessif.

5. Procédé pour élaborer des cellules différenciées qui sont homozygotes pour au moins un allèle pathogène récessif, consistant à :
a) activer des ovocytes non fertilisés dont l'ADN contient un allèle pathogène récessif ;
b) exposer les ovocytes activés à un traitement chimique qui inhibe l'extrusion du second globule polaire ;
c) cultiver les ovocytes exposés lors de l'étape (b) *in vitro* dans des conditions conduisant à la formation d'un blastocyste ;
d) cultiver des cellules d'embryoblaste du blastocyste *in vitro* dans des conditions conduisant à l'élaboration de cellules souches ;
e) cribler les cellules souches pour identifier des cellules souches homozygotes pour un allèle pathogène récessif ; et
f) différencier les cellules souches pluripotentes.

6. Procédé d'identification de molécules qui inhibent ou bloquent l'expression d'un gène pathogène récessif consistant à :
a) activer des ovocytes non fertilisés dont l'ADN contient un allèle pathogène récessif ;
b) exposer les ovocytes activés à un traitement chimique qui inhibe l'extrusion du second globule polaire ;
c) cultiver les ovocytes exposés lors de l'étape (b) *in vitro* dans des conditions conduisant à la formation d'un blastocyste ;
d) cultiver des cellules d'embryoblaste du blastocyste *in vitro* dans des conditions conduisant à l'élaboration de cellules souches ;
e) cribler les cellules souches pour identifier des cellules souches homozygotes pour un allèle pathogène récessif ;
f) différencier les cellules souches pluripotentes ; et
g) utiliser les cellules différenciées dans un test de criblage pour identifier des molécules qui inhibent ou bloquent l'expression dudit gène pathogène récessif, dans lequel le test de criblage est effectué in vitro.

7. Modèle animal non-humain pour étudier une maladie qui est provoquée par l'expression d'un gène pathogène récessif, qui est élaboré par l'introduction, dans un animal non-humain, des cellules différenciées à partir de cellules souches pluripotentes dérivées par parthénogenèse obtenues suivant le procédé selon la revendication 5, dans lequel le modèle animal non-humain est un modèle animal murin.
